(19) Europäisches Patentamt
European Patent Office
Office européen des brevets

(11) Numéro de publication : **0 578 516 A1**

(12) **DEMANDE DE BREVET EUROPEEN**

(21) Numéro de dépôt : **93401362.4**

(22) Date de dépôt : **28.05.93**

(51) Int. Cl.$^5$ : **C07D 233/86,** C07D 235/02, C07D 233/88

(30) Priorité : **08.07.92 FR 9208432**

(43) Date de publication de la demande : **12.01.94 Bulletin 94/02**

(84) Etats contractants désignés : **AT BE CH DE DK ES FR GB GR IT LI LU NL PT SE**

(71) Demandeur : **ROUSSEL-UCLAF
35, Boulevard des Invalides
F-75007 Paris (FR)**

(72) Inventeur : **Claussner, André
62, rue Marc Vieville
F-93250 Villemomble (FR)**
Inventeur : **Goubet, François
54, rue des Volontaires
F-75015 Paris (FR)**
Inventeur : **Teutsch, Jean-Georges
Résidence Lavoisier, Bât. 3, 3, rue Lavoisier
F-93500 Pantin (FR)**

(74) Mandataire : **Vieillefosse, Jean-Claude et al
ROUSSEL UCLAF Département des Brevets
111, Route de Noisy B.P. 9
F-93230 Romainville (FR)**

(54) **Nouvelles phénylimidazolidines éventuellement substituées, leur procédé de préparation, leur application comme médicaments et les compositions pharmaceutiques les renfermant.**

(57) L'invention a pour objet les produits de formule (I) :

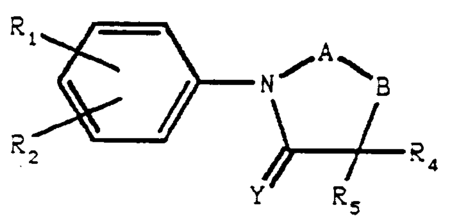

(I)

dans laquelle :
$R_1$ et $R_2$ identiques ou différents représentent cyano, nitro, halogène, trifluorométhyle ou carboxy libre, estérifié ou ramifié,
le groupement -A-B- est choisi parmi les radicaux

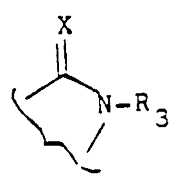

et

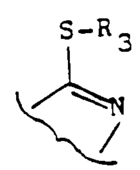

dans lesquels X représente oxygène ou soufre et $R_3$ est choisi parmi :
— hydrogène
— alkyle, alkényle, alkynyle, aryle ou aryl-alkyle, ces radicaux étant éventuellement substitués ;
Y représente oxygène ou soufre ou NH,
$R_4$ et $R_5$ identiques ou différents représentent hydrogène, alkyle éventuellement substitué ou $R_4$ et $R_5$ forment un cycloalkyle,
à l'exception des produits dans lesquels :
$R_4$ et $R_5$ représentent simultanément un radical méthyle, ceux dans lesquels l'un de $R_4$ ou $R_5$ représente un radical méthyle et l'autre un radical hydroxyméthyle, Y représente OH ou =NH et
-A-B- représente

EP 0 578 516 A1

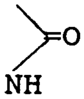

$R_1$ représente 4-$NO_2$ et $R_2$ représente 3-$CF_3$,
leur préparation, leur application comme médicaments notamment anti-androgènes.

La présente invention concerne de nouvelles phényl-imidazolidines éventuellement substituées, leur procédé de préparation, leur application comme médicaments et les compositions pharmaceutiques les renfermant.

Dans la demande japonaise J 48087030 sont décrites des 3-phényl 2-thiohydantoïnes qui sont présentées comme inhibant la germination de certaines plantes.

Dans le brevet français 2.329.276 sont décrites des imidazolidines qui sont présentées comme possédant une activité antiandrogène. Les produits de ce brevet sont cependant différents des produits de la présente demande de brevet.

La présente invention a donc pour objet les produits de formule générale (I) :

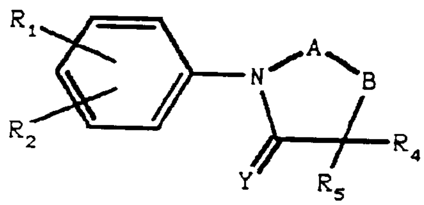

(I)

dans laquelle :

$R_1$ et $R_2$ identiques ou différents représentent un radical cyano, nitro, un atome d'halogène, un radical trifluorométhyle ou un radical carboxyle libre estérifié ou salifié,

le groupement -A-B- est choisi parmi les radicaux

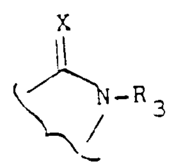

et

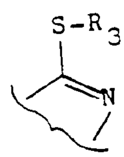

dans lesquels X représente un atome d'oxygène ou de soufre et $R_3$ est choisi parmi les radicaux suivants :

- un atome d'hydrogène,
- les radicaux alkyle, alkényle, alkynyle, aryle ou arylalkyle ayant au plus 12 atomes de carbone, ces radicaux étant éventuellement substitués par un ou plusieurs substituants choisis parmi les radicaux hydroxy, halogène, mercapto, cyano, acyle ou acyloxy ayant au plus 7 atomes de carbone, S-aryle éventuellement substitué, dans lequel l'atome de soufre est éventuellement oxydé sous forme de sulfoxyde ou de sulfone, carboxy libre, estérifié, amidifié ou salifié, amino, mono ou dialkylamino ou un radical hétérocyclique comprenant 3 à 6 chaînons et renfermant un ou plusieurs hétéroatomes choisis parmi les atomes de soufre, d'oxygène ou d'azote,

les radicaux alkyle, alkényle ou alkynyle étant de plus éventuellement interrompus par un ou plusieurs atomes d'oxygène, d'azote ou de soufre éventuellement oxydé sous forme de sulfoxyde ou de sulfone, les radicaux aryle et aralkyle étant de plus éventuellement substitués par un radical alkyle, alkényle ou alkynyle, alkoxy, alkényloxy, alkynyloxy ou trifluorométhyle,

Y représente un atome d'oxygène ou de soufre ou un radical =NH,

$R_4$ et $R_5$ identiques ou différents représentent un atome d'hydrogène, un radical alkyle ayant de 1 à 12 atomes de carbone éventuellement substitué par un ou plusieurs atomes d'halogène, de préférence le fluor ou $R_4$ et $R_5$ représentent ensemble avec l'atome de carbone auquel ils sont liés un radical cycloalkyle ayant de 3 à 7 atomes de carbone,

à l'exception des produits dans lesquels $R_4$ et $R_5$ représentent simultanément un radical méthyle et ceux dans lesquels l'un de $R_4$ ou $R_5$ représente un radical méthyle et l'autre représente un radical hydroxyméthyle, Y représente un atome d'oxygène ou un radical =NH, le groupement -A-B- représente le radical

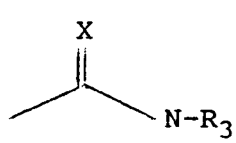

dans lequel X représente un atome d'oxygène et $R_3$ représente un atome d'hydrogène, $R_1$ en position 4 représente un radical nitro et $R_2$ en position 3 représente un radical trifluorométhyle.

Pour la définition des substituants indiqués ci-dessus et dans ce qui suit, les définitions utilisées peuvent avoir les valeurs suivantes :

Par alkyle ayant au plus 12 atomes de carbone on entend par exemple les valeurs méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle, heptyle, octyle, décyle, undécyle, dodécyle, linéaires ou ramifiés.

On préfère les radicaux alkyle ayant au plus 4 atomes de carbone et notamment les radicaux méthyle, éthyle, propyle, isopropyle.

Par alkényle ayant au plus 12 et préférentiellement 4 atomes de carbone on entend par exemple les valeurs suivantes:

- vinyle, allyle, 1-propényle, butényle, pentényle, hexenyle.

Parmi les valeurs alkényle, on préfère les valeurs allyle ou butényle.

Par alkynyle ayant au plus 12 et préférentiellement 4 atomes de carbone on entend par exemple les valeurs suivantes:

- éthynyle, propargyle, butynyle, pentynyle ou hexynyle.

Parmi les valeurs alkynyle, on préfère la valeur propargyle.

Par radical cycloalkyle ayant de 3 à 7 atomes de carbone, on entend par exemple, les groupements carbocycliques tel que cyclopropyle, cyclobutyle, cyclopentyle, cyclohexyle, cycloheptyle et cyclooctyle.

Par aryle on entend les radicaux aryles carbocyclique tels que phényle ou naphtyle ou les aryles hétérocycliques à 5 ou 6 chaînons comportant un ou plusieurs hétéroatomes choisis de préférence parmi l'oxygène, le soufre et l'azote. Parmi les aryles hétérocycliques à 5 chaînons on peut citer les radicaux furyle, thiényle, pyrrolyle, thiazolyle, oxazolyle, imidazolyle, thiadiazolyle, pyrazolyle, isoxazolyle.

Parmi les aryles hétérocycliques à 6 chaînons on peut citer les radicaux pyridyle, pyrimidinyle, pyridazinyle, pyrazinyle.

Parmi les radicaux aryles condensés on peut citer les radicaux indolyle, benzofurannyle, benzothiényle, quinoléïnyle.

On préfère le radical phényle.

Par arylalkyle on entend les radicaux résultant de la combinaison des radicaux alkyle cités précédemment et les radicaux aryle également cités ci-dessus.

On préfère les radicaux benzyle ou phényléthyle.

Par halogène, on entend bien entendu, les atomes de fluor, de chlore, de brome ou d'iode.

On préfère les atomes de fluor, de chlore ou de brome.

Comme exemples particuliers de radicaux alkyle substitués par un ou plusieurs halogènes, on peut citer les monofluoro, chloro, bromo ou iodométhyle, les difluoro, dichloro ou dibromométhyle, le trifluorométhyle.

Comme exemples particuliers de radicaux aryles ou aralkyles substitués, on peut citer ceux dans lesquels le radical phényle est substitué en position para, par un atome de fluor ou par un radical méthoxy ou trifluorométhyle.

Par radical acyle, on entend de préférence un radical ayant au plus 7 atomes de carbone tel que le radical acétyle, propionyle, butyryle ou benzoyle, mais peut également représenter un radical valéryle, hexanoyle, acryloyle, crotonoyle ou carbamoyle : on peut également citer le radical formyle.

Par radical acyloxy, on entend les radicaux dans lesquels les radicaux acyle ont la signification indiquée ci-dessus et par exemple les radicaux acétoxy ou propionyloxy.

Par carboxy estérifié on entend par exemple les radicaux tels que les radicaux alkyloxycarbonyle par exemple méthoxycarbonyle, éthoxycarbonyle, propoxycarbonyle, butyl ou tert-butyloxycarbonyle.

On peut également citer des radicaux formés avec les restes esters facilement clivables tels que les radicaux méthoxyméthyle, éthoxyméthyle ; les radicaux acyloxyalkyle tels que pivaloyloxyméthyle, pivaloyloxyéthyle, acétoxyméthyle ou acétoxyéthyle ; les radicaux alkyloxycarbonyloxy alkyle tels que les radicaux méthoxycarbonyloxy méthyle ou éthyle, les radicaux isopropyloxycarbonyloxy méthyle ou éthyle.

Une liste de tels radicaux esters peut-être trouvée par exemple dans le brevet européen EP 0 034 536.

Par carboxy amidifié on entend les radicaux du type

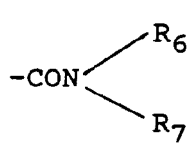

dans lesquels les radicaux $R_6$ et $R_7$ identiques ou différents représentent un atome d'hydrogène ou un radical alkyle ayant de 1 à 4 atomes de carbone tels que les radicaux méthyle, éthyle, propyle, isopropyle, butyle, isobutyle, sec-butyle ou tert-butyle.

Parmi les radicaux

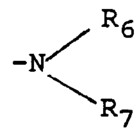

on préfère les radicaux amino, mono ou diméthylamino.

Le radical

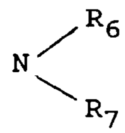

peut également représenter un hétérocycle qui peut ou non comporter un hétéroatome supplémentaire. On peut citer les radicaux pyrrolyle, imidazolyle, pyridyle, pyrazinyle, pyrimidyle, indolyle, pipéridino, morpholino, pipérazinyle. On préfère les radicaux pipéridino, ou morpholino.

Par carboxy salifié on entend les sels formés par exemple avec un équivalent de sodium, de potassium, de lithium, de calcium, de magnésium ou d'ammonium. On peut également citer les sels formés avec les bases organiques telles que la méthylamine, la propylamine, la triméthylamine, la diéthylamine, la triéthylamine.

On préfère le sel de sodium.

Par radical alkylamino on entend de préférence les radicaux dans lesquels l'alkyle comprend au plus 4 atomes de carbone. On peut citer les radicaux méthylamino, éthylamino, propylamino ou butyl (linéaire ou ramifié), amino.

De même, par radical dialkylamino on entend de préférence les radicaux dans lesquels l'alkyle comprend au plus 4 atomes de carbone. On peut citer par exemple les radicaux diméthylamino, diéthylamino, méthyléthylamino.

Par radical hétérocyclique renfermant un ou plusieurs hétéroatomes, on entend par exemple les radicaux monocycliques, hétérocycliques saturés tels que les radicaux oxirannyle, oxolannyle, dioxolannyle, imidazolidinyle, pyrazolidinyle, pipéridyle, pipérazinyle ou morpholinyle.

Par radicaux alkyle, alkényle, ou alkynyle éventuellement interrompus par un hétéroatome choisis parmi les atomes de soufre, d'oxygène ou d'azote, on entend les radicaux comprenant un ou plusieurs de ces atomes, identiques ou différents dans leur structure. Ces hétéroatomes ne pouvant évidemment pas être situés à l'extrémité du radical. On peut citer par exemple les radicaux alkoxyalkyle tels que méthoxyméthyle ou méthoxyéthyle ou encore les radicaux alkoxy alkoxyalkyle tels que méthoxyéthoxyméthyle.

Lorsque les produits de formule (I) comportent un radical amino salifiable par un acide il est bien entendu que ces sels d'acides font également partie de l'invention. On peut citer les sels formés avec les acides chlorhydrique ou méthanesulfonique par exemple.

L'invention a notamment pour objet les produits de formule (I) telle que définie ci-dessus dans laquelle Y représente un atome d'oxygène et le groupement -A-B- représente le radical :

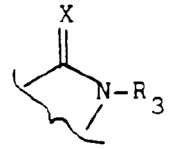

dans lequel X et $R_3$ ont les significations indiquées ci-dessus.

Parmi ces produits, l'invention a particulièrement pour objet ceux dans lequel le groupement -A-B- représente le groupement :

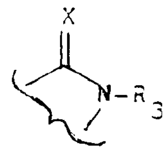

dans lequel X a la signification indiquée ci-dessus et $R_3$ représente un atome d'hydrogène ou un radical alkyle ayant de 1 à 6 atomes de carbone éventuellement substitué par un ou plusieurs radicaux choisis parmi les radicaux hydroxy éventuellement estérifié et carboxyle libre, estérifié ou salifié ou éventuellement interrompu par un ou plusieurs atomes d'oxygène ou de soufre.

Parmi ces produits, l'invention a particulièrement pour objet ceux dans lesquels $R_3$ représente un atome d'hydrogène ou un radical alkyle ayant au plus 6 atomes de carbone, de préférence 4 atomes de carbone, éventuellement substitué par un radical hydroxy.

Parmi ces produits, l'invention a plus particulièrement pour objet ceux dans lesquels $R_2$ en position 3, représente un radical trifluorométhyle et $R_1$ en position 4 représente un radical cyano.

Parmi ces produits, l'invention a tout particulièrement pour objet ceux dans lesquels $R_4$ et $R_5$ identiques ou différents représentent un atome d'hydrogène, un radical éthyle ou trifluorométhyle ou $R_4$ et $R_5$ forment ensemble avec l'atome de carbone auquel ils sont liés un radical cyclobutyle ou cyclopentyle.

Parmi les produits préférés de l'invention, on peut citer plus précisément les produits de formule (I) telle que définie ci-dessus dont les noms suivent :

- le 4-(3-méthyl 5-oxo 2-thioxo 1-imidazolidinyl) 2-(trifluorométhyl) benzonitrile,
- le 4-[1-méthyl 4-oxo 2-thioxo 1,3-diazaspiro (4,4)-nonan 3-yl] 2-(trifluorométhyl) benzonitrile,
- le 4-(4,4-diéthyl 3-méthyl 5-oxo 2-thioxo 1-imidazolidinyl) 2-(trifluorométhyl) benzonitrile.

L'invention a aussi pour objet un procédé de préparation des produits de formule générale (I) telle que définie ci-dessus caractérisé en ce que :

soit l'on fait agir en présence d'une base tertiaire un produit de formule (II) :

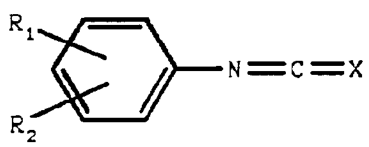

(II)

dans laquelle $R_1$, $R_2$ et X ont la signification indiquée ci-dessus, avec un produit de formule (III) :

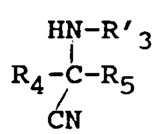

(III)

dans laquelle $R_4$ et $R_5$ ont la signification indiquée ci-dessus et $R'_3$ a les valeurs indiquées ci-dessus pour $R_3$ dans lequel les éventuelles fonctions réactives sont éventuellement protégées et étant entendu que $R_4$ et $R_5$ ne représentent pas simultanément un radical méthyle et que si $R_1$ représente un radical $NO_2$ en position 4, $R_2$ représente un radical $CF_3$ en position 3, X représente un atome d'oxygène et $R'_3$ représente un atome d'hydrogène, alors l'un de $R_4$ ou $R_5$ ne représente pas un radical $CH_3$ et l'autre un radical $CH_2OH$, pour obtenir un produit de formule (IV) :

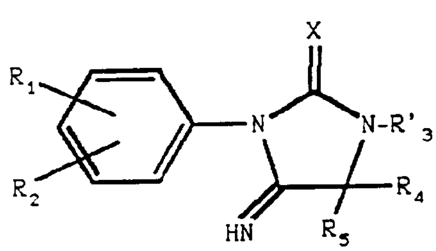

(IV)

dans laquelle $R_1$, $R_2$, X, $R'_3$, $R_4$ et $R_5$ ont la signification précédente, produits de formule (IV) que, si nécessaire ou si désiré l'on soumet à l'une quelconque ou plusieurs des réactions suivantes, dans un ordre quelconque :

a) réaction d'élimination des éventuels groupements protecteurs que peut porter $R'_3$ ;

b) réaction d'hydrolyse du groupement >C=NH en fonction cétone et le cas échéant transformation du groupement >C=S en groupement >C=O ;

c) réaction de transformation du ou des groupements >C=O en groupement >C=S ;

d) action sur les produits de formule (IV) dans laquelle $R'_3$ représente un atome d'hydrogène, et après hydrolyse du groupement >C=NH en fonction cétone d'un réactif de formule Hal-$R''_3$ dans laquelle $R''_3$ a les valeurs de $R'_3$ à l'exception de la valeur hydrogène et Hal représente un atome d'halogène pour obtenir des produits de formule (I) dans laquelle le groupement -A-B- représente le groupement

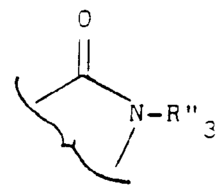

ou

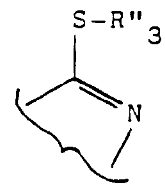

dans lesquels $R''_3$ a la signification indiquée précédemment puis, si désiré, action sur ces produits, d'un agent d'élimination des éventuels groupements protecteurs que peut porter $R''_3$ ou le cas échéant, action d'un agent d'estérification, d'amidification ou de salification,

soit l'on fait agir en présence d'une base tertiaire un produit de formule (II) :

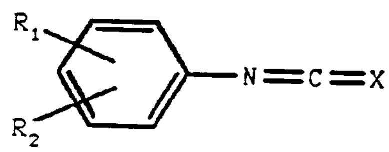

(II)

dans laquelle $R_1$, $R_2$ et X ont la signification indiquée ci-dessus, avec un produit de formule (III') :

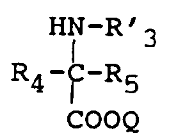

(III')

dans laquelle $R'_3$, $R_4$ et $R_5$ ont la signification indiquée ci-dessus et Q représente soit un atome de métal alcalin par exemple le sodium ou un radical alkyle renfermant de 1 à 6 atomes de carbone, pour obtenir pour obtenir un produit de formule (IVa) :

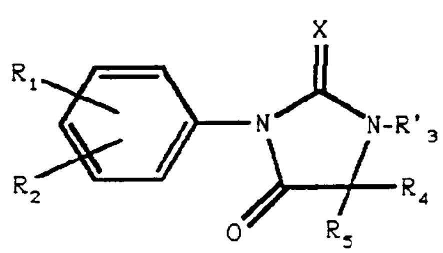

(IVa)

dans laquelle X, $R_1$, $R_2$, $R'_3$, $R_4$ et $R_5$ ont la signification indiquée ci-dessus, que si désiré l'on soumet à l'une quelconque ou plusieurs des réactions suivantes, dans un ordre quelconque :

a) réaction d'élimination des éventuels groupements protecteurs que peut porter $R'_3$ ;

b) réaction de transformation du ou des groupements >C=O en groupement >C=S ou le cas échéant du groupement >C=S en groupement >C=O ;

c) action sur les produits de formule (IVa) dans laquelle $R'_3$ représente un atome d'hydrogène, d'un réactif de formule Hal-$R''_3$ dans laquelle $R''_3$ a les valeurs de $R'_3$ à l'exception de la valeur hydrogène et Hal représente un atome d'halogène pour obtenir des produits de formule (I) dans laquelle le groupement -A-B-

représente le groupement

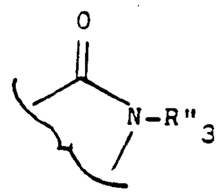

ou

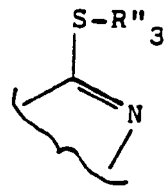

dans lesquels R"$_3$ a la signification indiquée précédemment puis, si désiré, action sur ces produits, d'un agent d'élimination des éventuels groupements protecteurs que peut porter R"$_3$ ou le cas échéant, action d'un agent d'estérification, d'amidification ou de salification,

soit l'on fait agir un réactif de formule Hal-R"$_3$ dans laquelle Hal et R"$_3$ ont les valeurs indiquées précédemment sur un produit de formule (IV') :

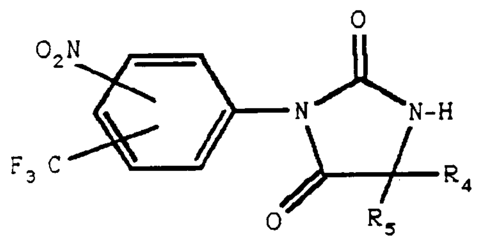

(IV')

pour obtenir un produit de formule (IV") :

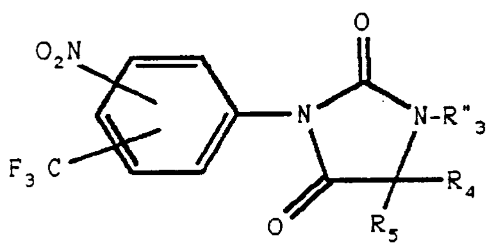

(IV")

produit de formule (IV") que, si nécessaire ou si désiré l'on soumet à l'une quelconque ou plusieurs des réactions suivantes dans un ordre quelconque :

a) réaction d'élimination des éventuels groupements protecteurs que peut porter R"$_3$ puis le cas échéant action d'un agent d'estérification, d'amidification ou de salification ;

b) réaction de transformation du ou des groupements >C=O en groupements >C=S.

L'action des produits de formule (II) avec les produits de formule (III) est effectuée de préférence dans un solvant organique tel que le tétrahydrofuranne ou le dichloroéthane mais on peut également utiliser l'éther éthylique ou l'éther isopropylique.

On opère en présence d'une base tertiaire telle que la triéthylamine ou encore la pyridine ou la méthyléthylpyridine.

Les éventuelles fonctions réactives que peut comporter R$_3$ et qui sont éventuellement protégées dans le produit de formule (III), (IVa) ou (IV") sont les fonctions hydroxy ou amino. On utilise pour protéger ces fonctions des groupements protecteurs usuels. On peut citer par exemples les groupements protecteurs suivants du radical amino : tert-butyle, tert-amyle, trichloroacétyle, chloroacétyle, benzhydryle, trityle, formyle, benzyloxycarbonyle.

Comme groupement protecteur du radical hydroxy on peut citer les radicaux tels que formyle, chloroacétyle, tétrahydropyrannyle, triméthylsilyle, tert-butyl diméthylsilyle.

Il est bien entendu que la liste ci-dessus n'est pas limitative et que d'autres groupements protecteurs, par exemple connus dans la chimie des peptides peuvent être utilisés. Une liste de tels groupements protecteurs se trouve par exemple dans le brevet français BF 2.499.995 dont le contenu est incorporé ici par référence.

Les réactions éventuelles d'élimination des groupements protecteurs sont effectuées comme indiqué dans ledit brevet BF 2.499.995. Le mode préféré d'élimination est l'hydrolyse acide à l'aide des acides choisis parmi les acides chlorhydrique, benzène sulfonique ou para toluène sulfonique, formique ou trifluoroacétique. On

préfère l'acide chlorhydrique.

La réaction éventuelle d'hydrolyse du groupement >C=NH en groupement cétone est également effectuée de préférence à l'aide d'un acide tel que l'acide chlorhydrique aqueux par exemple au reflux.

Lorsque l'hydrolyse du groupement >C=NH en groupement cétone est effectuée sur une molécule comportant également un groupement >C=S, celui-ci peut être transformé en groupement >C=O. Le radical OH libre que peut comporter éventuellement $R_3$ peut être alors transformé en radical SH.

La réaction de transformation du ou des groupements >C=O en groupement >C=S est effectuée à l'aide du réactif dit de Lawesson de formule :

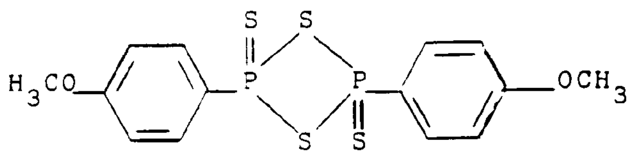

qui est un produit commercialisé par exemple par la firme FLUKA et dont l'utilisation est décrite par exemple dans la publication : Bull. Soc. Chim. Belg. vol 87, N° 3, (1987) p. 229.

Lorsque l'on veut transformer deux fonctions >C=O en deux fonctions >C=S on opère en présence d'un excès de réactif de Lawesson. Il en est de même lorsque l'on part d'une molécule comportant une fonction >C=S et une fonction >C=O et que l'on veut transformer ladite fonction >C=O en fonction >C=S.

Par contre lorsque l'on part d'une molécule comportant deux fonctions >C=O et que l'on veut obtenir un produit ne comportant qu'une seule fonction >C=S. On opère en présence d'un déficit de réactif de Lawesson. On obtient alors en général un mélange de trois produits : chacun des deux produits comportant une fonction >C=O et une fonction >C=S et le produit comportant deux fonctions >C=S. Ces produits peuvent être ensuite séparés par les méthodes usuelles telles que la chromatographie.

L'action sur les produits de formules (IV), (IVa) ou (IV') du réactif de formule Hal-$R''_3$ est effectuée en présence d'une base forte telle que l'hydrure de sodium ou de potassium. On peut opérer par réaction de transfert de phase en présence de sels d'ammonium quaternaires tels que le tert-butyl ammonium.

- Les groupements protecteurs que peut porter le substituant $R''_3$ pouvant être par exemple un de ceux précédemment cités pour $R_3$. Les réactions d'élimination des groupements protecteurs s'effectuent dans les conditions indiquées ci-dessus.

Un exemple d'élimination du groupement terbutyldiméthylsilyle au moyen de l'acide chlorhydrique est donné ci-après dans les exemples.

- L'estérification éventuelle des produits de formule (I) dans laquelle $R''_3$ comporte un radical OH libre est effectuée dans des conditions classiques. On peut utiliser par exemple un acide ou un dérivé fonctionnel, par exemple un anhydride tel que l'anhydride acétique en présence d'une base telle que la pyridine.

L'estérification ou la salification éventuelle des produits de formule (I) dans laquelle $R''_3$ représente un groupement COOH est effectuée dans les conditions classiques connues de l'homme du métier.

- L'amidification éventuelle des produits de formule (I) dans laquelle $R''_3$ comporte un radical COOH est effectuée dans des conditions classiques. On peut utiliser une amine primaire ou secondaire sur un dérivé fonctionnel de l'acide par exemple un anhydride symétrique ou mixte.

La présente invention a également pour objet un procédé de préparation des produits de formule (I") :

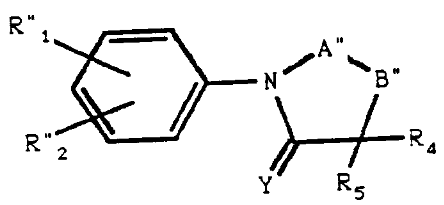

(I")

dans laquelle $R''_1$, $R''_2$, $R_4$, $R_5$, -A"-B"- ont les significations indiquées ci-dessus pour $R_1$, $R_2$ et -A-B- étant entendu que lorsque -A"-B"- représente un groupement -CO-N($R'''_3$)- dans lequel $R'''_3$ représente un atome d'hydrogène ou un radical alkyle linéaire ou ramifié ayant au plus 7 atomes de carbone et Y représente un atome d'oxygène, $R''_1$ représente un radical cyano, ce procédé étant caractérisé en ce que l'on fait réagir un produit de formule (V) :

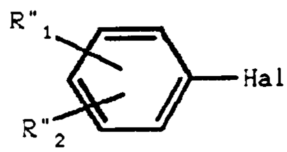

(V)

dans laquelle R"$_1$ et R"$_2$ ont les significations précédentes et Hal représente un atome d'halogène avec un produit de formule (VI) :

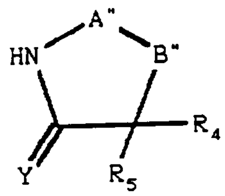

(VI)

dans laquelle -A"-B"-, $R_4$, $R_5$ et Y ont la signification indiquée ci-dessus, la réaction s'effectuant en présence d'un catalyseur et éventuellement d'un solvant.

En ce qui concerne les produits de formule (V), le terme Hal désigne de préférence l'atome de chlore, mais peut aussi représenter un atome de brome ou d'iode.

Le rôle du catalyseur est vraisemblablement de piéger l'halogénure d'hydrogène qui se dégage et ainsi de faciliter la réaction de condensation du produit de formule (V) avec le produit de formule (VI) pour donner le produit recherché.

L'invention a plus précisément pour objet un procédé tel que défini ci-dessus dans lequel le catalyseur est un métal sous forme native ou oxydée ou une base.

Quand le catalyseur utilisé est un métal, ce métal peut être du cuivre ou du nickel. Il peut être sous forme native, sous forme d'oxyde métallique ou encore sous forme de sels métalliques.

Les sels métalliques peuvent être un chlorure ou un acétate.

Quand le catalyseur est une base, cette base peut être par exemple la soude ou la potasse et on peut, si désiré, ajouter au milieu réactionnel du diméthylsulfoxyde.

L'invention a plus précisément pour objet un procédé tel que défini ci-dessus dans lequel le catalyseur est choisi parmi l'oxyde cuivreux, l'oxyde cuivrique, le cuivre sous forme native et une base telle que la soude ou la potasse.

Le cuivre sous forme native utilisé comme catalyseur est préférentiellement sous forme de poudre.

L'invention a particulièrement pour objet un procédé tel que défini ci-dessus dans lequel le catalyseur est l'oxyde cuivreux.

Le solvant utilisé est préférentiellement choisi parmi des éthers à haut point d'ébullition tels que, par exemple, l'oxyde de phényle, le diglyme, le triglyme et le diméthylsulfoxyde mais peut être également, par exemple, une huile à haut point d'ébullition telle que la paraffine ou la vaseline.

L'invention a plus particulièrement pour objet un procédé tel que défini ci-dessus caractérisé en ce que l'on opère en présence d'un solvant de type éther tel que l'oxyde de phényle, le diglyme, le triglyme ou le diméthylsulfoxyde.

L'invention a tout particulièrement pour objet un procédé tel que défini ci-dessus dans lequel le solvant utilisé est l'oxyde de phényle ou le triglyme.

Le procédé de préparation du produit recherché défini ci-dessus peut être réalisé sous pression ou à la pression atmosphérique, à une température préférentiellement élevée.

L'invention a ainsi pour objet un procédé tel que défini ci-dessus caractérisé en ce que la réaction est réalisée à une température supérieure à 100°C et de préférence supérieure à 150°C.

L'invention a plus précisément pour objet un procédé tel que défini ci-dessus caractérisé en ce que la réaction est réalisée pendant plus de 2 heures.

L'invention a très précisément pour objet un procédé tel que défini ci-dessus caractérisé en ce que la réaction est réalisée en présence d'oxyde cuivreux, dans le triglyme, à une température supérieure ou égale à 200°C et pendant plus de 3 heures.

Les produits objets de la présente invention sont doués de propriétés pharmacologiques intéressantes ; on a constaté notamment qu'ils inhibaient les effets des androgènes sur les récepteurs périphériques.

Des tests donnés dans la partie expérimentale illustrent cette activité anti-androgène.

Du fait de cette activité anti-androgène, les produits de l'invention peuvent être utilisés en thérapeutique

chez les adultes sans avoir à redouter certains effets d'une castration chimique.

Ces propriétés rendent les produits de formule générale (I) de la présente invention utilisables comme médicaments pour le traitement des adénomes et des néoplasies de la prostate ainsi que pour lutter contre l'hypertrophie bénigne de la prostate.

Ces propriétés rendent les produits de formule générale (I) également utilisables dans le traitement des tumeurs bénignes ou malignes dont les cellules contiennent notamment des récepteurs androgènes. On peut en particulier citer principalement les cancers du sein, du cerveau, de la peau et des ovaires mais également les cancers de la vessie, du système lymphatique, du rein, du foie.

Les produits de formule générale (I) de l'invention trouvent également leur utilisation dans le traitement de l'hirsutisme, de l'acné, de la seborrhée, de l'alopécie androgénique, de l'hyperpilosité ou hirsutisme.

Les produits de formule (I) peuvent donc être utilisés en dermatologie : ils peuvent être utilisés seuls ou en association. Ils peuvent être associés notamment avec un produit antibiotique tels que les dérivés de l'acide azélaique, fusidique, l'érythromycine ou avec un dérivé des rétinoïdes pour le traitement de l'acné, ou avec un inhibiteur de la 5α-réductase tel que le (5α,17β)-1,1-diméthyléthyl 3-oxo 4-aza-androst-l-ène 17-carboxamide (ou Finastéride Merck, 11ème ed.) ou l'acide azélaïque ou un agent bloquant des récepteurs androgènes pour le traitement de l'acné, de l'alopécie ou de l'hirsutisme, ou avec un produit stimulant la croissance des cheveux tel que le Minoxidil pour le traitement de l'alopécie.

Les produits de formule (I) peuvent également être utilisés dans le domaine vétérinaire.

Les produits de formule (I), sous forme de produits radioactifs peuvent encore être utilisés en diagnostic comme marqueurs spécifiques des récepteurs androgènes. Comme produits radioactifs, on peut utiliser par exemple des produits marqués au tritium, au carbone 14 ou encore à l'iode 125.

L'invention a donc pour objet l'application, à titre de médicaments, des produits de formule générale (I) pharmaceutiquement acceptables.

L'invention a particulièrement pour objet l'application à titre de médicaments, des produits dont les noms suivent :

- le 4-(3-méthyl 5-oxo 2-thioxo 1-imidazolidinyl) 2-(trifluorométhyl) benzonitrile,
- le 4-[1-méthyl 4-oxo 2-thioxo 1,3-diazaspiro (4,4)-nonan 3-yl] 2-(trifluorométhyl) benzonitrile,
- le 4-(4,4-diéthyl 3-méthyl 5-oxo 2-thioxo 1-imidazolidinyl) 2-(trifluorométhyl) benzonitrile.

Les produits peuvent être administrés par voie parentérale, buccale, perlinguale, rectale ou topique.

L'invention a aussi pour objet les compositions pharmaceutiques, caractérisées en ce qu'elles renferment, à titre de principe actif, un au moins des médicaments de formule générale (I).

Ces compositions peuvent être présentées sous forme de solutions ou de suspensions injectables, de comprimés, de comprimés enrobés, de capsules, de sirops, de suppositoires, de crèmes, de pommades et de lotions. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, le beurre de cacao, les corps gras d'origine animale ou végétale, les dérivés paraffiniques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'homme, par voie orale.

Les produits de formule (II) utilisés au départ de l'invention peuvent être obtenus par action du phosgène lorsque X représente un atome d'oxygène ou du thiophosgène lorsque X représente un atome de soufre sur l'amine correspondante de formule (A) :

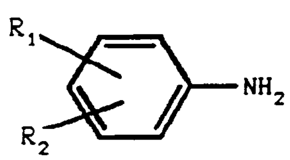

(A)

Un exemple d'une telle préparation est donné ci-après dans la partie expérimentale. Un produit de ce type est décrit également dans le brevet français BF 2.329.276.

Les amines de formule (A) sont décrites dans le brevet européen EP 0.002.892 ou le brevet français BF 2.142.804.

Les produits de formule (III) ou (III') sont connus ou peuvent être préparés à partir de la cyanhydrine correspondante selon le procédé décrit dans les publications : J. Am. Chem. Soc. (1953), 75, 4841, BEIL I 4 526 ou J. Org. Chem. 27 2901 (1962).

Les produits de formule (III) dans lesquels $R'_3$ est différent d'un atome d'hydrogène peuvent être obtenus

par action d'un produit de formule R"$_3$ Hal sur le 2-cyano 2-amino propane dans les conditions énoncées ci-dessus pour l'action de R"$_3$ Hal sur les produits de formule (IV). Un exemple de préparation de ce type est décrit dans la référence :

- Jilek et Coll. Collect. Czech. Chem. Comm. 54(8) 2248 (1989).

Les produits de formule (IV') sont décrits dans le brevet français BF 2.329.276.

Les produits de départ de formules (V) et (VI), sur lesquels s'exerce un procédé, objet de l'invention, pour l'obtention des produits de formule (I), sont connus et disponibles dans le commerce ou peuvent être préparés selon des méthodes connues de l'homme de métier.

La préparation de produits de formule (VI) est décrite notamment dans les publications suivantes :

- Zhur. Préklad. Khim. 28, 969-75 (1955) (CA 50, 4881a, 1956)
- Tétrahédron 43, 1753 (1987)
- J. Org. 52, 2407 (1987)
- Zh. Org. Khim. 21, 2006 (1985)
- J. Fluor. Chem. 17, 345 (1981)

ou dans les brevets :

- allemand DRP 637.318 (1935)
- européen EP 0.130.875
- japonais JP 81.121.524.

Les produits de formule (VI) qui sont des dérivés de l'hydantoïne sont largement utilisés et cités dans la littérature comme par exemple dans les articles suivants :

- J. Pharm. Pharmacol., 67, Vol. 19(4), p. 209-16 (1967)
- Khim. Farm. Zh., 67, Vol. 1 (5) p. 51-2
- Brevet allemand 2.217.914
- Brevet européen 0.091.596
- J. Chem. Soc. Perkin. Trans. 1, p. 219-21 (1974).

L'invention a également pour objet, à titre de produits industriels nouveaux et notamment à titre de produits industriels nouveaux utilisables comme intermédiaires pour la préparation des produits de formule générale (I), les produits de formule (IVi) :

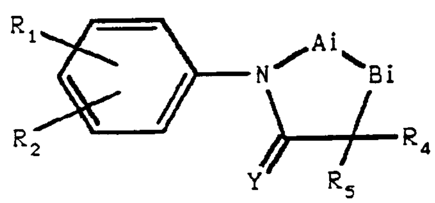

(IVi)

dans laquelle $R_1$, $R_2$ et Y ont les significations indiquées ci-dessus et le groupement :

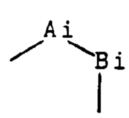

est choisi parmi les radicaux :

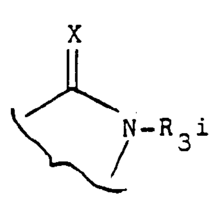

et

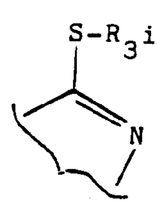

dans lesquels X représente un atome d'oxygène ou de soufre et $R_{3i}$ est choisi parmi les valeurs de $R_3$ comportant une fonction réactive protégée.

Parmi les fonctions réactives qui peuvent être protégées on peut citer les fonctions hydroxyle et amino. Ces fonctions peuvent être protégées comme indiqué ci-dessus pour le substituant $R_3$.

Les exemples suivants illustrent l'invention sans toutefois la limiter.

**EXEMPLE 1 : 4-(3-méthyl 5-oxo 2-thioxo 1-imidazolidinyl) 2-(trifluorométhyl) benzonitrile**

a) A une solution de 22 $cm^3$ d'eau distillée et 1 $cm^3$ de thiophosgène on ajoute lentement 2,23 g de 2-trifluorométhyl 4-amino benzonitrile (préparé selon EP 0002892) on agite pendant 1 heure, extrait avec du chloroforme, lave à l'eau salée, sèche et évapore à sec sous pression réduite, on obtient 3 g de produit utilisé tel quel.

b) A 976 mg de N-méthylglycyne en solution dans 3,65 $cm^3$ de soude (3 M/l) on ajoute 2,5 g d'isothiocyanate préparé ci-dessus, en solution dans 5 $cm^3$ d'éthanol. On agite 30 minutes à température ambiante puis porte au reflux pendant 1 heure. On laisse revenir à température ambiante, verse sur un mélange de 20 $cm^3$ d'eau et 10 $cm^3$ d'acide chlorhydrique N et extrait au chloroforme.

Après chromatographie sur silice (éluant chlorure de méthylène-acétone (95-5)), on obtient 1,78 g de produit que l'on recristallise dans un mélange chlorure de méthylènecyclohexane. On recueille 1,66 g de produit attendu.

F = 220-221°C.

rf = 0,18 cyclohexane-acétate d'éthyle 1-1.

Spectre IR ($CHCl_3$)

| | |
|---|---|
| C=O | 1788-1729 $cm^{-1}$ |
| C≡N | 2235 $cm^{-1}$ |
| système conjugué + Aromatiques | 1614-1580-1515 $cm^{-1}$ |

Spectre UV (EtOH)

Max. 232 nm ε = 17300

Max. 254 nm ε = 22700.

**EXEMPLE 2 : 4-(1-méthyl 4-imino 2-thioxo 1,3-diazaspiro(4,4)nonan 3-yl) 2-(trifluorométhyl) benzonitrile**

On ajoute en 2 minutes environ, 1,36 g de 1-méthylamino cyclopentane carbonitrile en solution dans 10 $cm^3$ de tétrahydrofuranne dans 2,5 g de l'isothiocyanate préparé comme à l'exemple 1 stade a).

On agite 40 minutes, évapore le solvant, chromatographie le résidu sur silice (éluant chlorure de méthylène-acétate d'éthyle (87,5-12,5)) et obtient 3,32 g de produit attendu.

F = 165-166°C.

rf = 0,3 (chlorure de méthylène-acétate d'éthyle (85-15))

Spectre IR ($CHCl_3$)

=NH 3310-1672 $cm^{-1}$

C≡N 2230 $cm^{-1}$

Aromatiques 1614-1577-1505 $cm^{-1}$

**Préparation du 1-méthylamino cyclopentane carbonitrile** utilisé au départ de l'exemple 2.

On refroidit à 15-20°C 8,5 g de cyclopentanone, 7 g de chlorhydrate de méthylamine dans 7,5 $cm^3$ d'eau et ajoute 6,5 g de cyanure de potassium dans 13 $cm^3$ d'eau. On laisse revenir à température ambiante et agite 18 heures, extrait au chlorure de méthylène. On lave la phase organique à l'eau salée, sèche et évapore le solvant. On distille le résidu et obtient 4,1 g de produit attendu. Eb : 60° ± 0,3°C (sous 7 mm de Hg).

**EXEMPLE 3 : 4-(1-méthyl 4-oxo 2-thioxo 1,3-diazaspiro (4,4)nonan 3-yl) 2-(trifluorométhyl) benzonitrile**

On dissout dans 5,2 $cm^3$ de chloroforme 259 mg de produit préparé à l'exemple 2, puis ajoute 52 $cm^3$ de méthanol. A la solution obtenue, on ajoute 7,5 $cm^3$ d'acide chlorhydrique 2N puis chauffe 1 heure au reflux. On refroidit à température ambiante et verse sur 150 $cm^3$ d'eau glacée. On extrait au chloroforme, lave la phase organique à l'eau salée, sèche et évapore le solvant. On chromatographie le résidu sur silice (acétate d'éthyle-cyclohexane (3-7)), recueille les fractions rf = 0,35 et obtient après cristallisation dans le chlorure de méthylène

et cyclohexane 247 mg de produit attendu.
F = 162-163°C.
Rf = 0,35 (éluant cyclohexane-acétate d'éthyle (7-3)).
Spectre IR ($CHCl_3$)

| | |
|---|---|
| C=O | 1765 $cm^{-1}$ |
| C≡N | 2235 $cm^{-1}$ |
| Aromatiques | 1609-1578-1505 $cm^{-1}$ |

Spectre UV (EtOH)

| | |
|---|---|
| Max. 234 nm | ε = 17600 |
| Max. 256 nm | ε = 23800 |
| inf. 266 nm | ε = 20300. |

**<u>EXEMPLE 4</u> : 4-(4,4-diéthyl 3-méthyl 5-imino 2-thioxo 1-imidazolidinyl 2-(trifluorométhyl) benzonitrile**

En opérant comme à l'exemple 2, en utilisant au départ 2,5 g d'isothiocyanate préparé comme à l'exemple 1 stade a) et 1,39 g de l'aminonitrile approprié, on obtient 3,22 g de produit attendu. F = 167-168°C.
rf = 0,27 (chlorure de méthylène-acétate d'éthyle (85-15))
Spectre IR ($CHCl_3$)

| | |
|---|---|
| =NH | 3304-1673 $cm^{-1}$ |
| C≡N | 2230 $cm^{-1}$ |
| Aromatiques | 1614-1576-1505 $cm^{-1}$ |

**Préparation du 1-méthylamino diéthyl carbonitrile** utilisé au départ de l'exemple 4.

On opère comme pour la préparation du 1-méthylamino cyclopentane carbonitrile donnée à l'exemple 2, en utilisant au départ 8,6 g de diéthyl cétone. On obtient 4,8 g de produit attendu. Eb = 77°C (sous 40 mm de Hg).

**<u>EXEMPLE 5</u> : 4-(4,4-diéthyl 3-méthyl 5-oxo 2-thioxo 1-imidazolidinyl 2-(trifluorométhyl) benzonitrile**

En opérant comme à l'exemple 3, à partir de 321 mg de produit obtenu à l'exemple 4 dans 65 $cm^3$ de méthanol et 14 $cm^3$ d'acide chlorhydrique 2N, on obtient 249 mg de produit attendu. F = 126-127°C.
Rf = 0,45 (éluant cyclohexane-acétate d'éthyle (4-6)).
Spectre IR ($CHCl_3$)

| | |
|---|---|
| C=O | 1753 $cm^{-1}$ |
| C≡N | 2235 $cm^{-1}$ |
| Aromatiques | 1615-1580-1504 $cm^{-1}$ |

Spectre UV (EtOH)

| | |
|---|---|
| Max. 234 nm | ε = 17800 |
| Max. 254 nm | ε = 24100 |
| inf. 265 nm | |

**<u>EXEMPLE 6</u> : 4-(5-méthyl 8-imino 6-thioxo) 5,7-diazaspiro (3,4) octan 7-yl) 2-trifluorométhyl benzonitrile.**

Dans 456 mg d'isothiocyanate préparé comme à l'exemple 1 stade a), dans 2 $cm^3$ de 1,2-dichloroéthane, on ajoute en 3 minutes, 221 mg de 1-méthylamino cyclobutane carbonitrile en solution dans 1 $cm^3$ de 1,2-dichloroéthane en présence de 0,2 $cm^3$ de triéthylamine. On agite pendant 45 minutes, évapore le solvant, chromatographie le résidu sur silice (éluant : chlorure de méthylène-acétone 95-5) et récupère les fractions de rf = 0,32. Après cristallisation dans l'éther, on obtient 610 mg de produit attendu. F = 172-173°C.
Spectre IR ($CHCl_3$)

| | |
|---|---|
| >C=NH | 3315-1673 $cm^{-1}$ |
| C≡N | 2236 $cm^{-1}$ |
| Aromatiques | 1615-1580-1505 $cm^{-1}$ |

**Préparation du 1-méthylamino cyclobutane carbonitrile.**

On opère comme indiqué à la préparation du 1-méthylamino cyclopentane carbonitrile décrite à l'exemple 2 en utilisant au départ 7 g de cyclobutanone. On obtient 10,6 g de produit attendu.

**EXEMPLE 7 : 4-(5-méthyl 8-oxo 6-thioxo 5,7-diazaspiro (3,4) octan-7-yl) 2-(trifluorométhyl) benzonitrile.**

On opère comme à l'exemple 3 en utilisant au départ 514 mg de produit obtenu à l'exemple 6 et 1,5 $cm^3$ d'acide chlorydrique 2N. Après chromatographie sur silice (cyclohexaneacétate d'éthyle 6-4), on récupère les fractions de rf = 0,34 et obtient après cristallisation dans l'éther 499 mg de produit attendu. F = 161-162°C.

Spectre IR ($CHCl_3$)

| | |
|---|---|
| >C=O | 1754 $cm^{-1}$ |
| C≡N | 2236 $cm^{-1}$ |
| Aromatiques | 1615-1583-1504 $cm^{-1}$ |

Spectre UV (EtOH)

| | |
|---|---|
| Inf. 239 nm | $\varepsilon$ = 17400 |
| Max. 257 nm | $\varepsilon$ = 21200 |
| inf. 268 nm | $\varepsilon$ = 19000 |

**EXEMPLE 8 : 4-(1-méthyl 4-imino 2-oxo 1,3-diazaspiro (4,4) nonan-3-yl) 2-(trifluorométhyl) benzonitrile.**

On refroidit à -3°C, 300 mg de 1-méthylamino cyclopentane carbonitrile dans 3 $cm^3$ de 1,2-dichloroéthane en présence de 0,5 $cm^3$ de triéthylamine. On ajoute en 1 minute 1,5 $cm^3$ d'une solution d'isocyanate de 3-trifluorométhyl 4-benzonitrile préparé comme indiqué à l'exemple 1a) à partir de phosgène et de 2-trifluorométhyl 4-aminobenzonitrile (1,6M/l) dans le 1,2-dichloroéthane. On agite 40 minutes, évapore le solvant et obtient après chromatographie sur silice (éluant : chlorure de méthylène-acétate d'éthyle 95-5) 620 mg de produit attendu.

**EXEMPLE 9 : 4-(1-méthyl 2,4-dioxo 1,3-diazaspiro (4,4) nonan-3-yl) 2-(trifluorométhyl) benzonitrile.**

On chauffe à 50°C 535 mg de produit obtenu ci-dessus en solution dans 10 $cm^3$ de méthanol et 2 $cm^3$ d'acide chlorhydrique 2N. On agite 1 heure, ramène à température ambiante, ajoute 20 $cm^3$ d'eau et extrait au chlorure de méthylène. Après élimintation du solvant sous pression réduite, on dissout le résidu dans l'acétone, chromatographie le résidu sur silice (éluant : chlorure de méthylène-acétone 98-2) et obtient 325 mg de produit attendu.

Spectre IR ($CHCl_3$)

| | |
|---|---|
| C=O | 1777 (m), 1724 $cm^{-1}$ (F) |
| C≡N | 2238 $cm^{-1}$ |
| Aromatiques | 1616-1576-1505 $cm^{-1}$ |

Spectre UV (EtOH)

| | |
|---|---|
| Inf. 236 nm | $\varepsilon$ = 10000 |
| Max. 262 nm | $\varepsilon$ = 13900 |
| inf. 277 nm | $\varepsilon$ = 7200 |
| inf. 286 nm | $\varepsilon$ = 3700 |

**EXEMPLE 10 : 4-(5-méthyl 8-imino 6-oxo 5,7-diazaspiro (3,4) octan-7-yl) 2-(trifluorométhyl) benzonitrile.**

On opère comme à l'exemple 8 en utilisant au départ 2 $cm^3$ de la solution d'isocyanate et 352 mg de 1-méthylamino cyclobutane carbonitrile. Après chromatographie sur silice (éluant 85-15), on recueille les fractions de rf = 0,20 et obtient 301 mg de produit attendu. F = 144-145°C.

Spectre IR ($CHCl_3$)

| | |
|---|---|
| OH/NH | 3295 $cm^{-1}$ |
| C≡N | 2240 $cm^{-1}$ |
| >C=O | 1740 $cm^{-1}$ |
| C=N | 1664 $cm^{-1}$ |

Aromatiques 1611-1572-1508 $cm^{-1}$

**EXEMPLE 11 : 4-(6,8-dioxo 5-méthyl 5,7-diazaspiro (3,4) octan-7-yl) 2-(trifluorométhyl) benzonitrile.**

On opère comme à l'exemple 9 à partir de 0,8 g de produit obtenu comme à l'exemple 10 et 3 $cm^3$ d'acide chlorhydrique 2N en utilisant le chloroforme comme solvant d'extraction. Après chromatographie sur silice (éluant : chlorure de méthylène-acétate d'éthyle 95-5), on obtient 465 mg de produit attendu. F = 165-166°C.
Spectre IR ($CHCl_3$)
C≡N 2236 $cm^{-1}$
C=O 1778, 1726 $cm^{-1}$
Aromatiques 1616, 1579, 1505 $cm^{-1}$
Spectre UV (EtOH)
Max. 238 nm ε = 11000
Max. 262 nm ε = 14000
inf. 278-286 nm

**EXEMPLE 12 : 4-(4-imino 2-oxo 1,3-diazaspiro (4,4) nonan-3-yl) 2-(trifluorométhyl) benzonitrile.**

On opère comme à l'exemple 8 en utilisant au départ 3,1 $cm^3$ de la solution 1,6M d'isocyanate et 550 mg de 1-amino cyclopentane carbonitrile. Après chromatographie sur silice (éluant : chlorure de méthylène-acétone 90-10), on recueille 1,24 g de produit attendu. F = 212-213°C.
Spectre IR ($CHCl_3$)
OH/NH 3350, 3290 $cm^{-1}$
C≡N 2240 $cm^{-1}$
C=O 1744 $cm^{-1}$
>=N 1678 $cm^{-1}$
Aromatiques 1610-1574-1510 $cm^{-1}$

**Préparation du 1-amino cyclopentane carbonitrile.**

On refroidit à O°/+8°C, 7,9 g de chlorure d'ammonium et 6,14 g de cyanure de sodium dans 40 $cm^3$ d'ammoniaque puis ajoute goutte à goutte 8,8 $cm^3$ de cyclopentane. On laisse revenir à température ambiante et agite 16 heures. On extrait au chlorure de méthylène, lave la phase organique à l'eau salée, sèche et évapore le solvant à une température inférieure à 30°C. On distille le résidu et obtient 11 g de produit attendu. Eb : 55° ± 2° (sous 11 mg de Hg).

**EXEMPLE 13 : 4-(2,4-dioxo 1,3-diazaspiro (4,4) nonan-3-yl) 2(trifluorométhyl) benzonitrile.**

On opère comme à l'exemple 9 à partir de 1,17 g de produit obtenu à l'exemple 12 et 5 $cm^3$ d'acide chlorhydrique 2N. Après chromatographie sur silice (éluant : chlorure de méthylène-acétone 9-1), on obtient 1,108 g de produit attendu. rf = 0,23. F = 184-185°C.
Spectre IR ($CHCl_3$)
=C-NH 3444 $cm^{-1}$
C≡N 2296 $cm^{-1}$
>=O 1786, 1731 $cm^{-1}$
Aromatiques 1616, 1505 $cm^{-1}$
Spectre UV (EtOH)
Max. 258 nm ε = 15600
Max. 286 nm ε = 3500

**EXEMPLE 14 : 4-(8-imino 6-oxo 5,7-diazaspiro (3,4) octan-7-yl) 2-(trifluorométhyl) benzonitrile.**

En opérant comme à l'exemple 8 en utilisant au départ 3,1 $cm^3$ de la solution d'isocyanate et 480 mg de 1-amino cyclobutane carbonitrile, on obtient 990 mg de produit attendu. rf = 0,25. F = 192-193°C.
Spectre IR ($CHCl_3$)
OH/NH 3380, 3315 $cm^{-1}$
C≡N 2240 $cm^{-1}$
>=N 1754 $cm^{-1}$

Aromatiques 1612, 1571, 1510 $cm^{-1}$

Le 1-amino cyclobutane carbonitrile a été préparé comme indiqué à la préparation du 1-amino cyclopentane carbonitrile (exemple 12) en utilisant au départ 7,4 $cm^3$ de cyclobutanone. On obtient 9,2 g de produit attendu.

**EXEMPLE 15 : 4-(6,8-dioxo 5,7-diazaspiro (3,4) octan-7-yl) 2-(trifluorométhyl) benzonitrile.**

On opère comme à l'exemple 9 en utilisant au départ 372 mg de produit obtenu à l'exemple 14 (en ajoutant 1,5 $cm^3$ de chloroforme pour obtenir la solubilisation du produit) et 1,9 $cm^3$ d'acide chlorhydrique 2N. Après chromatographie sur silice (éluant : chlorure de méthylène-acétone 9-1), on obtient 341 mg de produit attendu. rf = 0,32. F = 210-211°C.

Spectre IR ($CHCl_3$)

| | |
|---|---|
| OH/NH | 3390 $cm^{-1}$ |
| C≡N | 2240 $cm^{-1}$ |
| C=O | 1787, 1737 $cm^{-1}$ |
| Aromatiques | 1612, 1577, 1508 $cm^{-1}$ |

Spectre UV (EtOH)

Max. 259 nm $\varepsilon$ = 15700

inf. 277, 286, 301 nm

**EXEMPLE 16 : 4-[1-(4-hydroxybutyl) 2,4-dioxo 1,3-diazaspiro (4,4) nonan-3-yl) 2-(trifluorométhyl) benzonitrile.**

On ajoute goutte à goutte en 35 minutes 808 mg de produit obtenu à l'exemple 13 en solution dans 7 $cm^3$ de diméthylformamide dans 142 mg d'hydrure de sodium. On ajoute, 10 minutes après cessation du dégagement d'hydrogène, 650 mg de 4-chloro terbutyldiméthylsilyléther et 408 mg d'iodure de sodium puis chauffe 3 heures à 70°C. On ramène à température ambiante, ajoute 71 mg d'hydrure de sodium supplémentaire, agite 10 minutes puis ajoute 330 mg de réactif silylé et 222 mg d'iodure de sodium et chauffe 45 minutes à 70°C. On laisse revenir à température ambiante, ajoute 60 $cm^3$ d'eau contenant environ 500 mg de phosphate monopotassique puis extrait à l'éther éthylique puis à l'acétate d'éthyle. On réunit les phases organiques, les sèche, évapore le solvant sous pression réduite, chromatographie le résidu sur silice (éluant : chlorure de méthylène-acétone 98-2) et obtient 560 mg d'intermédiaire silylé. On reprend 550 mg de cet intermédiaire dans 6 $cm^3$ de méthanol et 1,5 $cm^3$ d'acide chlorhydrique 2N. On agite 30 minutes, ajoute 30 $cm^3$ d'eau, extrait au chlorure de méthylène, sèche et évapore le solvant. Après chromatographie du résidu sur silice (éluant : chlorure de méthylène-acétone 9-1), puis cristallisation dans l'éther isopropylique, on obtient 381 mg de produit attendu. F = 125-126°C. rf = 0,17.

Spectre IR ($CHCl_3$)

| | |
|---|---|
| OH | 3625 $cm^{-1}$ |
| C≡N | 2235 $cm^{-1}$ |
| >=O | 1773, 1721 $cm^{-1}$ |
| Aromatiques | 1615, 1580, 1505 $cm^{-1}$ |

Spectre UV (EtOH)

Max. 239 nm $\varepsilon$ = 9800

Max. 262 nm $\varepsilon$ = 14600

inf. 286 nm

**EXEMPLE 17 : 4-[5-(4-hydroxybutyl) 6,8-dioxo 5,7-diazaspiro (3,4) octan-7-yl) 2-(trifluorométhyl) benzonitrile.**

En opérant comme à l'exemple 16, en utilisant au départ le produit obtenu à l'exemple 15, on obtient le produit attendu. F = 92-93°C.

Spectre IR ($CHCl_3$)

| | |
|---|---|
| OH | 3626 $cm^{-1}$ |
| C≡N | 2235 $cm^{-1}$ |
| >=O | 1775 (m), 1784 (F) $cm^{-1}$ |
| Aromatiques | 1616, 1578, 1505 $cm^{-1}$ |

Spectre UV (EtOH)

Max. 238 nm $\varepsilon$ = 11200

Max. 262 nm ε = 13900
inf. 288 nm

En opérant comme dans les exemples précédents à partir de l'isocyanate ou du thioisocyanate approprié et du 3,3,3-trifluoro 2-trifluoro méthyl 2-(méthylamino) propionitrile préparé comme indiqué dans J. Org. Chem. 35, 1485 (1970), on obtient les composés suivants :

**EXEMPLE 18 : 4-(4,4-bis trifluorométhyl 3-méthyl 5-imino 2-thioxo 1-imidazolidinyl) 2-(trifluorométhyl) benzonitrile.**

**EXEMPLE 19 : 4-(4,4-bis trifluorométhyl 3-méthyl 5-oxo 2-thioxo 1-imidazolidinyl) 2-(trifluorométhyl) benzonitrile.**

**EXEMPLE 20 : 4-(4,4-bis trifluorométhyl 3-méthyl 5-imino 2-oxo 1-imidazolidinyl) 2-(trifluorométhyl) benzonitrile.**

**EXEMPLE 21 : 4-(4,4-bis trifluorométhyl 3-méthyl 2,5-dioxo 1-imidazolidinyl) 2-(trifluorométhyl) benzonitrile.**

En plus des produits décrits dans les exemples qui illustrent l'invention, sans toutefois la limiter, les produits suivants constituent des produits pouvant être obtenus dans le cadre de la présente invention ; les substituants $R_1$, $R_2$, $R_3$, $R_4$, $R_5$, X et Y sont ceux indiqués dans la formule suivante :

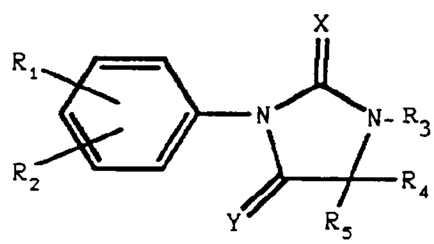

| $R_1$ | $R_2$ | X | Y | $R_4$ $R_5$ |
|---|---|---|---|---|
| C≡N | $CF_3$ | S | O | 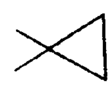 |
| " | " | O | " | " |
| " | " | S | " | 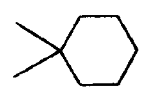 |
| " | " | O | " | " |

**EXEMPLE 22 :**

On a préparé des comprimés ayant la composition suivante:

- Produit de l'exemple 3 100 mg
- Excipient q.s. pour un comprimé terminé à 300 mg

(Détail de l'excipient : lactose, amidon, talc, stéarate de magnésium).

## ETUDE PHARMACOLOGIQUE DES PRODUITS DE L'INVENTION

1) Etude de l'affinité des produits de l'invention pour le récepteur androgène

**Récepteur androgène.**

Des rats mâles Sprague Dawley EOPS de 180-200 g, castrés de 24 heures, sont sacrifiés, les prostates prélevées, pesées et homogénéisées à 0°C à l'aide d'un potter verre-verre, dans une solution tamponnée (Tris 10mM, saccharose 0,25M, PMSF (phénylméthanesulfonylfluoride) 0,1mM, Molybdate de sodium 20mM, HCl pH 7,4 ; auxquels on ajoute extemporanément 2mM de DTT (DL dithiothreitol), à raison de 1 g de tissu pour 8 ml de tampon.

L'homogénat est ensuite ultracentrifugé à 0°C, 30 minutes à 209 000 g. Des aliquotes du surnageant obtenu (=cytosol), sont incubées 30 minutes et 24 heures à 0°C, avec une concentration constante (T) de Testostérone tritiée et en présence de concentrations croissantes (0 à $2500.10^{-9}$M), soit de testostérone froide, soit des produits à tester. La concentration de Testostérone tritiée liée (B) est ensuite mesurée dans chaque incubat par la méthode d'adsorption au charbon-dextran.

**Calcul de l'affinité relative de liaison (ARL).**

On trace les 2 courbes suivantes : le pourcentage de l'hormone tritiée liée B/T en fonction du logarithme de la concentration de l'hormone de référence froide et B/T en fonction du logarithme de la concentration du produit froid testé. On détermine la droite d'équation $I_{50}$=(B/Tmax + B/Tmin)/2.
B/T max= % de l'hormone tritiée liée pour une incubation de cette hormone tritiée à la concentration (T).
B/T min= % de l'hormone tritiée liée pour une incubation de cette hormone tritiée à la concentration (T) en présence d'un grand excès d'hormone froide ($2500.10^{-9}$M).

Les intersections de la droite $I_{50}$ et des courbes, permettent d'évaluer les concentrations de l'hormone de référence froide (CH) et du produit froid testé (CX) qui inhibent de 50 % la liaison de l'hormone tritiée sur le récepteur. L'affinité relative de liaison (ARL) du produit testé est déterminé par l'équation ARL=100 (CH)/(CX).

On obtient les résultats suivants exprimés en ARL.

| Produit de référence (Testostérone) : 100 | |
|---|---|
| Produit des exemples | Incubation : 24 heures |
| 3 | 27 |
| 5 | 8 |

2) Détermination de l'activité androgène ou anti-androgène des produits de l'invention à l'aide du dosage de l'ornithine décarboxylase.

**- Protocole de traitement**

Des souris mâles SWISS âgées de 6 semaines, et castrées de 24 heures, reçoivent par voie orale ou percutanée les produits à étudier (suspension en méthyl cellulose à 0,5 % ou en solution dans l'éthanol), simultanément avec une injection sous-cutanée de Propionate de testostérone 3 mg/kg (solution dans l'huile de maïs) pour déterminer l'activité anti-androgène. L'activité agoniste est déterminée en l'absence de propionate de testostérone.

Le Propionate de testostérone est administré sous un volume de 10 ml/kg.

20 heures après les traitements, les animaux sont sacrifiés, les reins prélevés, puis homogénéisés à 0°C , à l'aide d'un broyeur téflon-verre dans 10 volumes de tampon Tris-HCl 50 mM (pH 7,4) contenant 250 uM de phosphate de pyridoxal, 0,1 mM EDTA, et 5 mM de dithiothreitol. L'homogenat est ensuite centrifugé à 209000 g pendant 30 mn.

**- Principe de dosage**

A 37°C, l'ornithine décarboxylase rénale transforme un mélange isotopique d'ornithine froide et d'ornithine tritiée en putrescine froide et putrescine tritiée.

La putrescine est ensuite recueillie sur des papiers sélectifs, échangeurs d'ions. Après séchage, l'excès d'ornithine tritiée et froide non transformée est éliminé, par 3 lavages d'ammoniaque 0,1 M. Les papiers sont séchés, puis la radioactivité est comptée après addition de scintillant Aqualite.

Les résultats sont exprimés en fmoles ($10^{-15}$ M) de putrescine tritiée formée/heure/mg de protéines.

Les résultats sont exprimés en % d'inhibition de l'ODL des témoins ne recevant que le propionate de testostérone.

**Test :** les produits sont administrés par voie percutanée à 1,5 mg/kg sous un volume de 10 µl.

| Produits des exemples | Test |
|---|---|
| 3 | 28 |
| 5 | 43 |

**Conclusion :** Les tests indiqués ci-dessus montrent que les produits de l'invention testés possèdent une forte activité anti-androgène et sont dénués d'activité agoniste.

**Revendications**

**1)** Les produits de formule générale (I) :

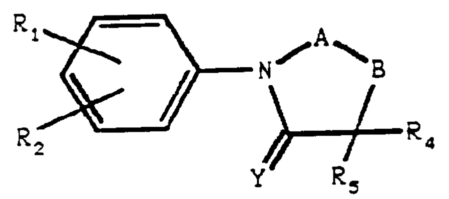

(I)

dans laquelle :
$R_1$ et $R_2$ identiques ou différents représentent un radical cyano, nitro, un atome d'halogène, un radical trifluorométhyle ou un radical carboxyle libre estérifié ou salifié, le groupement -A-B- est choisi parmi les radicaux

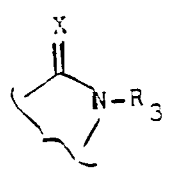

et

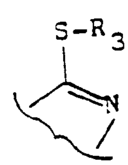

dans lesquels X représente un atome d'oxygène ou de soufre et $R_3$ est choisi parmi les radicaux suivants :

- un atome d'hydrogène,
- les radicaux alkyle, alkényle, alkynyle, aryle ou arylalkyle ayant au plus 12 atomes de carbone, ces radicaux étant éventuellement substitués par un ou plusieurs substituants choisis parmi les radicaux hydroxy, halogène, mercapto, cyano, acyle ou acyloxy ayant au plus 7 atomes de carbone, S-aryle éventuellement substitué, dans lequel l'atome de soufre est éventuellement oxydé sous forme de sulfoxyde ou de sulfone, carboxy libre, estérifié, amidifié ou salifié, amino, mono ou dialkylamino ou un radical hétérocyclique comprenant 3 à 6 chaînons et renfermant un ou plusieurs hétéroatomes choisis parmi les atomes de soufre, d'oxygène ou d'azote,

  les radicaux alkyle, alkényle ou alkynyle étant de plus éventuellement interrompus par un ou plusieurs atomes d'oxygène, d'azote ou de soufre éventuellement oxydé sous forme de sulfoxyde ou de sulfone, les radicaux aryle et aralkyle étant de plus éventuellement substitués par un radical alkyle, alkényle ou alkynyle, alkoxy, alkényloxy, alkynyloxy ou trifluorométhyle,

  Y représente un atome d'oxygène ou de soufre ou un radical =NH,

  $R_4$ et $R_5$ identiques ou différents représentent un atome d'hydrogène, un radical alkyle ayant de 1 à 12 atomes de carbone éventuellement substitué par un ou plusieurs atomes d'halogène, de préférence le fluor ou $R_4$ et $R_5$ représentent ensemble avec l'atome de carbone auquel ils sont liés un radical cycloalkyle ayant de 3 à 7 atomes de carbone, à l'exception des produits dans lesquels $R_4$ et $R_5$ représentent simultanément un radical méthyle et ceux dans lesquels l'un de $R_4$ ou $R_5$ représente un radical méthyle et l'autre représente un radical hydroxyméthyle, Y représente un atome d'oxygène ou un radical =NH, le groupement -A-B- représente le radical

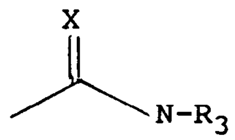

dans lequel X représente un atome d'oxygène et $R_3$ représente un atome d'hydrogène, $R_1$ en position 4 représente un radical nitro et $R_2$ en position 3 représente un radical trifluorométhyle.

**2)** Les produits de formule (I) telle que définie à la revendication 1, dans laquelle Y représente un atome d'oxygène, et le groupement -A-B- représente le radical :

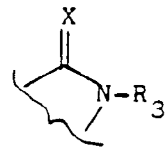

dans lequel X et $R_3$ ont les significations indiquées à la revendication 1.

**3)** Les produits de formule (I) telle que définie à la revendication 1 ou 2 dans laquelle le groupement -A-B- représente le groupement :

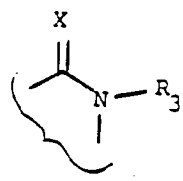

dans lequel X a la signification indiquée à la revendication 1 et $R_3$ représente un atome d'hydrogène ou un radical alkyle ayant de 1 à 6 atomes de carbone éventuellement substitué par un ou plusieurs radicaux choisis parmi les radicaux hydroxy éventuellement estérifié et carboxyle libre, estérifié ou salifié ou éventuellement interrompu par un ou plusieurs atomes d'oxygène ou de soufre.

**4)** Les produits de formule (I) selon la revendication 3, dans laquelle $R_3$ représente un atome d'hydrogène ou un radical alkyle ayant au plus 6 atomes de carbone, de préférence 4 atomes de carbone, éventuellement substitué par un radical hydroxy.

**5)** Les produits de formule (I) telle que définie à l'une quelconque des revendications 1 à 4, dans laquelle $R_2$ en position 3 représente un radical trifluorométhyle et $R_1$ en position 4 représente un radical cyano.

**6)** Les produits de formule (I) telle que définie à l'une quelconque des revendications 1 à 5, dans laquelle $R_4$ et $R_5$ identiques ou différents représentent un atome d'hydrogène, un radical éthyle ou trifluorométhyle ou $R_4$ et $R_5$ forment ensemble avec l'atome de carbone auquel ils sont liés un radical cyclobutyle ou cyclopentyle.

**7)** Les produits de formule (I) telle que définie à l'une quelconque des revendications 1 à 6, dont les noms suivent :

- le 4-(3-méthyl 5-oxo 2-thioxo 1-imidazolidinyl) 2-(trifluorométhyl) benzonitrile,
- le 4-[1-méthyl 4-oxo 2-thioxo 1,3-diazaspiro (4,4)-nonan 3-yl] 2-(trifluorométhyl) benzonitrile,
- le 4-(4,4-diéthyl 3-méthyl 5-oxo 2-thioxo 1-imidazolidinyl) 2-(trifluorométhyl) benzonitrile.

**8)** Procédé de préparation des produits de formule générale (I) telle que définie à la revendication 1, caractérisé en ce que :

soit l'on fait agir en présence d'une base tertiaire un produit de formule (II) :

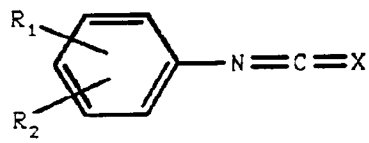

(II)

dans laquelle $R_1$, $R_2$ et X ont la signification indiquée ci-dessus, avec un produit de formule (III) :

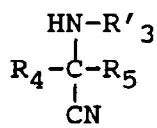

(III)

dans laquelle $R_4$ et $R_5$ ont la signification indiquée ci-dessus et $R'_3$ a les valeurs indiquées ci-dessus pour $R_3$ dans lequel les éventuelles fonctions réactives sont éventuellement protégées et étant entendu que $R_4$ et $R_5$ ne représentent pas simultanément un radical méthyle et que si $R_1$ représente un radical $NO_2$ en position 4, $R_2$ représente un radical $CF_3$ en position 3, X représente un atome d'oxygène et $R'_3$ représente un atome d'hydrogène, alors l'un de $R_4$ ou $R_5$ ne représente pas un radical $CH_3$ et l'autre un radical $CH_2OH$, pour obtenir un produit de formule (IV) :

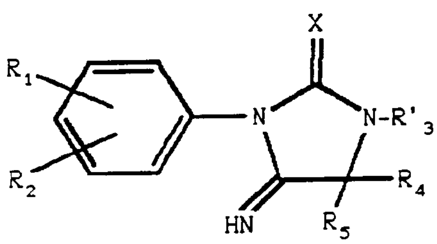

(IV)

dans laquelle $R_1$, $R_2$, X, $R'_3$, $R_4$ et $R_5$ ont la signification précédente, produits de formule (IV) que, si nécessaire ou si désiré l'on soumet à l'une quelconque ou plusieurs des réactions suivantes, dans un ordre quelconque :

a) réaction d'élimination des éventuels groupements protecteurs que peut porter $R'_3$ ;

b) réaction d'hydrolyse du groupement >C=NH en fonction cétone et le cas échéant transformation du groupement >C=S en groupement >C=O ;

c) réaction de transformation du ou des groupements >C=O en groupement >C=S ;

d) action sur les produits de formule (IV) dans laquelle $R'_3$ représente un atome d'hydrogène, et après hydrolyse du groupement >C=NH en fonction cétone d'un réactif de formule Hal-$R''_3$ dans laquelle $R''_3$ a les valeurs de $R'_3$ à l'exception de la valeur hydrogène et Hal représente un atome d'halogène pour obtenir des produits de formule (I) dans laquelle le groupement -A-B- représente le groupement

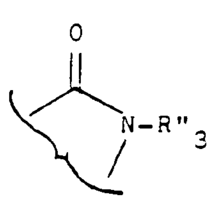

ou

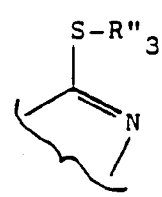

dans lesquels $R''_3$ a la signification indiquée précédemment puis, si désiré, action sur ces produits, d'un agent d'élimination des éventuels groupements protecteurs que peut porter $R''_3$ ou le cas échéant, action d'un agent d'estérification, d'amidification ou de salification,

soit l'on fait agir en présence d'une base tertiaire un produit de formule (II) :

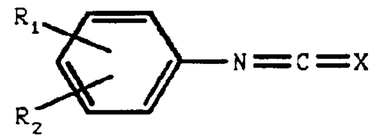

(II)

dans laquelle $R_1$, $R_2$ et X ont la signification indiquée ci-dessus, avec un produit de formule (III') :

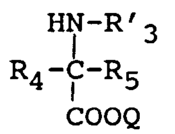

(III')

dans laquelle $R'_3$, $R_4$ et $R_5$ ont la signification indiquée ci-dessus et Q représente soit un atome de métal alcalin ou un radical alkyle renfermant de 1 à 6 atomes de carbone, pour obtenir pour obtenir un produit de formule (IVa) :

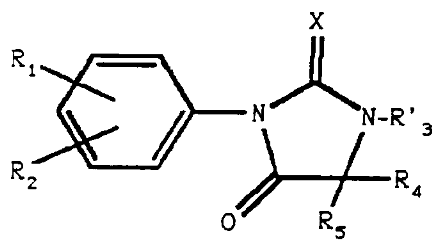

(IVa)

dans laquelle X, $R_1$, $R_2$, $R'_3$, $R_4$ et $R_5$ ont la signification indiquée ci-dessus, que si désiré l'on soumet à l'une quelconque ou plusieurs des réactions suivantes, dans un ordre quelconque :

a) réaction d'élimination des éventuels groupements protecteurs que peut porter $R'_3$ ;

b) réaction de transformation du ou des groupements >C=O en groupement >C=S ou le cas échéant du groupement >C=S en groupement >C=O ;

c) action sur les produits de formule (IVa) dans laquelle $R'_3$ représente un atome d'hydrogène, d'un réactif de formule Hal-$R''_3$ dans laquelle $R''_3$ a les valeurs de $R'_3$ à l'exception de la valeur hydrogène et Hal représente un atome d'halogène pour obtenir des produits de formule (I) dans laquelle le groupement -A-B- représente le groupement

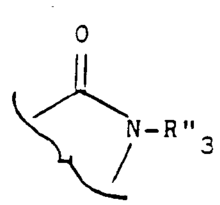

ou

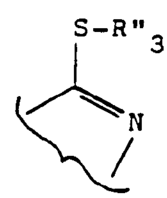

dans lesquels $R''_3$ a la signification indiquée précédemment puis, si désiré, action sur ces produits, d'un agent d'élimination des éventuels groupements protecteurs que peut porter $R''_3$ ou le cas échéant, action d'un agent d'estérification, d'amidification ou de salification,

soit l'on fait agir un réactif de formule Hal-$R''_3$ dans laquelle Hal et $R''_3$ ont les valeurs indiquées précédemment sur un produit de formule (IV') :

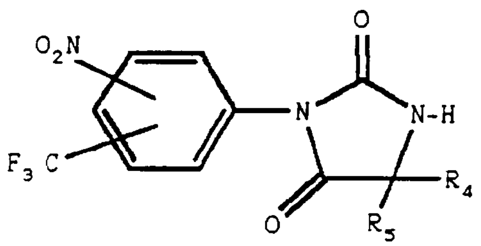

(IV')

pour obtenir un produit de formule (IV") :

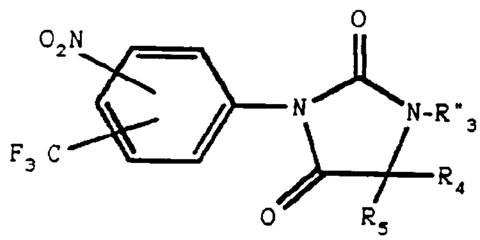

(IV")

produit de formule (IV") que, si nécessaire ou si désiré l'on soumet à l'une quelconque ou plusieurs des réactions suivantes dans un ordre quelconque :

a) réaction d'élimination des éventuels groupements protecteurs que peut porter R"$_3$ puis le cas échéant action d'un agent d'estérification, d'amidification ou de salification ;

b) réaction de transformation du ou des groupements >C=O en groupements >C=S.

**9)** A titre de médicaments, les produits de formule (I) tels que définis aux revendications 1 à 6, pharmaceutiquement acceptables.

**10)** A titre de médicaments, les produits de formule (I) tels que définis à la revendication 7.

**11)** Les compositions pharmaceutiques contenant, à titre de principe actif, l'un au moins des médicaments tels que définis à l'une quelconque des revendications 9 et 10.

**12)** A titre de produits industriels nouveaux, les produits de formule (IVi) :

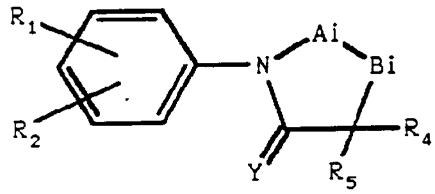

(IVi)

dans laquelle $R_1$, $R_2$, $R_4$, $R_5$ et Y ont les significations indiquées à la revendication 1 et le groupement :

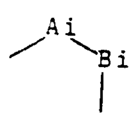

est choisi parmi les radicaux :

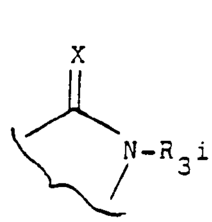

et

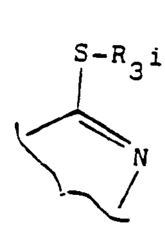

dans lesquels X représente un atome d'oxygène ou de soufre et $R_{3i}$ est choisi parmi les valeurs de $R_3$ comportant une fonction réactive protégée.

Office européen des brevets

# RAPPORT DE RECHERCHE EUROPEENNE

Numero de la demande
EP 93 40 1362

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | EP-A-0 091 596 (CELAMERCK)<br>*Document complet*<br>--- | 1-12 | C07D233/86<br>C07D235/02<br>C07D233/88 |
| A | EP-A-0 001 813 (HOFFMANN-LA ROCHE)<br>*Document complet*<br>--- | 1-12 | |
| A | EP-A-0 436 426 (ROUSSEL-UCLAF)<br>*Document complet*<br>--- | 1-12 | |
| A | FR-A-2 075 751 (SUMITOMO)<br>*Document complet*<br>--- | 1-12 | |
| A | US-A-4 473 393 (KRISHEN L. NAGPAL)<br>*Document complet*<br>--- | 1-12 | |
| A | US-A-4 753 957 (HAK-FOON CHAN)<br>*Document complet*<br>--- | 1-12 | |
| A | US-A-B379038 (ARTHUR MAGNANI)<br>*Document complet*<br>--- | 1-12 | DOMAINES TECHNIQUES RECHERCHES (Int.Cl.5) |
| A | CHEMICAL ABSTRACTS, vol. 81, no. 7, 19 Août 1974, Columbus, Ohio, US;<br>abstract no. 34559b,<br>page 149 ;colonne L ;<br>* abrégé * | 1-12 | C07D |
| D,A | & JP-A-7 387 030 (SUMITOMO)<br>--- | 1-12 | |
| D,A | FR-A-2 329 276 (ROUSSEL-UCLAF)<br>*Document complet*<br>--- | 1-12 | |
| P,X | EP-A-0 494 819 (ROUSSEL-UCLAF)<br>*Document complet*<br>----- | 1-12 | |

Le présent rapport a été établi pour toutes les revendications

| Lieu de la recherche | Date d'achèvement de la recherche | Examinateur |
|---|---|---|
| LA HAYE | 21 Octobre 1993 | LUYTEN, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)